# DEMANDE DE BREVET EUROPEEN

(11) **EP 4 485 950 A1**
(43) Date de publication de la demande: **01.01.2025**
(21) Numéro de dépôt: 24184111.3
(22) Date de dépôt: 24.06.2024
(51) Int. Cl.: H04N 21/45, H04N 21/466, H04N 21/482, H04N 21/442

(54) **PROCEDE ET DECODEUR AUDIOVISUEL POUR LA RECOMMANDATION D'UNE LISTE D'AU MOINS UN PROGRAMME AUDIOVISUEL**

(30) Priorité: 26.06.2023 FR 2306657
(71) Demandeur: Sagemcom Broadband SAS, 92270 Bois-Colombes (FR)
(72) Inventeur: INDIVERI, Piero, 92270 BOIS-COLOMBES (FR); SCHOTT, Vincent, 92270 BOIS-COLOMBES (FR); BERGER, Jérôme, 92270 BOIS-COLOMBES (FR)
(74) Mandataire: Cabinet Le Guen Maillet

(57) **Abrégé**

La présente invention concerne un procédé et un décodeur audiovisuel (10) pour la recommandation d'une liste d'au moins un programme audiovisuel à au moins un utilisateur, le ou les programmes étant catégorisés en genres et/ou sous-genres. Selon l'invention, le procédé (100) comprend les étapes exécutées par ledit décodeur audiovisuel (10) de :
- association (E20) desdits genres/sous-genres à des états émotionnels ;
- détection (E40) d'un état émotionnel courant dudit au moins un utilisateur ;
- réalisation d'un tri (E60) desdits programmes dudit catalogue tenant au moins compte dudit état émotionnel détecté et desdits genres et/ou sous-genres associés audit état émotionnel détecté ;
- affichage (E80) d'une liste d'au moins un programme audiovisuel tenant compte dudit tri desdits programmes dudit catalogue.

De cette manière, l'invention permet de prendre en compte l'état émotionnel courant détecté de l'utilisateur afin d'améliorer la pertinence des programmes qui lui sont recommandés.

## Description

### DOMAINE TECHNIQUE

Le domaine technique de l'invention est celui de la diffusion de contenus ou de programmes audiovisuels, notamment. La présente invention concerne plus particulièrement un procédé de recommandation d'un programme ou d'une liste de programmes audiovisuels tenant compte de l'émotion de l'utilisateur.

### ETAT DE LA TECHNIQUE ANTERIEURE

Les plateformes de distribution de programmes (notamment audiovisuels) disposent de catalogues de programmes de plus en plus étendus. Il est donc parfois difficile pour l'utilisateur de rechercher et sélectionner un programme qui lui convient. Pour faciliter cette sélection, les plateformes mettent généralement en oeuvre des procédés de recommandation de programmes tenant compte notamment de la catégorie des programmes et du nombre de lectures de ces programmes de sorte à proposer à l'utilisateur les programmes les plus populaires de chaque catégorie.

Cependant, cette technique n'est pas satisfaisante puisqu'elle ne prend pas en compte l'état émotionnel courant de l'utilisateur et les programmes qui lui sont proposés ne sont donc pas nécessairement en phase avec les attentes de l'utilisateur à cet instant. En effet, selon l'état émotionnel de l'utilisateur, ses préférences peuvent différer et ne pas correspondre aux recommandations effectuées classiquement par les plateformes de distribution.

Il existe donc un besoin de fournir un procédé de recommandation d'un programme, notamment audiovisuel, à un utilisateur tenant notamment compte de son état émotionnel afin d'améliorer la pertinence des programmes qui lui sont proposés.

### EXPOSE DE L'INVENTION

À cette fin, selon un premier aspect, l'invention propose un procédé de recommandation d'une liste d'au moins un programme audiovisuel parmi un catalogue de programmes à au moins un utilisateur orienté vers un décodeur, en particulier un décodeur audiovisuel, et des moyens d'affichage, lesdits programmes dudit catalogue étant catégorisés en genres et/ou sous-genres.

On entend par catalogue, dans l'ensemble de cette description, une base de programmes disponibles à la lecture par un utilisateur, ici via un décodeur audiovisuel.

Il est à noter que la présente invention est décrite en particulier en relation avec la lecture de programmes audiovisuels. On comprend aisément que la présente solution pourrait être mise en oeuvre pour la recommandation de programmes de type audio et/ou visuels, comme des podcasts, des livres audio, de la musique, etc. ou des photos, notamment. Ainsi, on entend par lecture, le fait qu'un utilisateur maintient pendant une durée prédéterminée la reproduction, par le décodeur audiovisuel 10, d'un programme, quel que soit le type du programme.

Pour des questions de clarté et de compréhension de l'invention, on décrit ci-après que les programmes du catalogue sont catégorisés par genres. On entend ici qu'un genre (par exemple film/drame) peut comprendre une pluralité de sous-genres (par exemple Aventure/western/guerre, Science-fiction/fantastique/horreur, Comédie, etc.) ou que le genre peut en fait être, dans une alternative, un sous-genre.

L'invention concerne aussi un décodeur audiovisuel apte à mettre un tel procédé de recommandation.

Ainsi, la présente invention permet de prendre en compte l'état émotionnel courant détecté d'au moins un utilisateur afin d'améliorer la pertinence de la liste d'au moins un programme qui lui est recommandée.

Selon un aspect particulier de l'invention, le procédé comporte les étapes exécutées par ledit décodeur audiovisuel de :
- association desdits genres à des états émotionnels ;
- détection d'un état émotionnel courant dudit au moins un utilisateur ;
- réalisation d'un tri desdits programmes dudit catalogue de programmes tenant au moins compte dudit état émotionnel détecté desdits genres associés audit état émotionnel détecté ;
- affichage d'une liste d'au moins un programme audiovisuel tenant compte dudit tri desdits programmes dudit catalogue de programmes.

Selon un mode particulier de l'invention, ladite étape d'association desdits genres auxdits états émotionnels comprend une sous-étape d'utilisation d'une table de priorité dans laquelle, pour chacun desdits états émotionnels, lesdits genres sont classés selon un ordre de priorité, ledit classement tenant au moins compte d'un compteur de lectures de chaque genre associé à chaque état émotionnel, ladite table de priorité comprenant en outre un genre par défaut attribué en dernier selon l'ordre de priorité pour chacun desdits états émotionnels.

Selon un aspect particulier de ce mode particulier, ladite étape de réalisation d'un tri desdits programmes dudit catalogue tenant au moins compte dudit état émotionnel détecté et desdits genres associés audit état émotionnel détecté comprend une sous-étape de :
- pointage, dans ladite table de priorité, dudit genre classé en premier selon ledit ordre de priorité pour ledit état émotionnel détecté, et
- détection d'une sélection par l'utilisateur et si l'utilisateur ne sélectionne pas un programme correspondant audit premier genre pointé, pointage du genre suivant dans la table de priorité jusqu'à ce que l'utilisateur sélectionne un programme dudit genre pointé ou jusqu'à ce que ledit genre pointé soit ledit genre par défaut.

Selon un aspect particulier de ce mode particulier, ladite étape d'affichage de ladite liste d'au moins un programme audiovisuel tenant compte dudit tri desdits programmes dudit catalogue comprend les sous-étapes de :
- ordonnancement dudit au moins un programme de ladite liste d'au moins un programme à afficher, ledit ordonnancement tenant au moins en partie compte d'un critère de priorité dudit au moins un programme ; et
- affichage de ladite liste d'au moins un programme en fonction dudit ordonnancement.

Selon un autre mode particulier de l'invention, ladite étape d'association desdits genres auxdits états émotionnels comprend une sous-étape d'utilisation d'une table de comptage comprenant un compteur de lectures par l'utilisateur pour chaque genre associé à un état émotionnel.

Selon un aspect particulier de cet autre mode particulier, ladite étape de réalisation d'un tri desdits programmes dudit catalogue tenant au moins compte dudit état émotionnel détecté et desdits genres associés audit état émotionnel détecté comprend les sous-étapes de :
- calcul d'un coefficient de pondération pour chacun desdits genres associés audit état émotionnel détecté ;
- pour chaque programme compris dans une liste de programmes proposés dans laquelle chaque programme présente un score prédéterminé et un genre, calcul d'un score pondéré par la multiplication dudit score prédéterminé dudit programme par ledit coefficient de pondération correspondant audit genre;
- hiérarchisation desdits programmes de ladite liste de programmes proposés par ordre décroissant de score pondéré.

Selon un aspect particulier de cet autre mode particulier, la sous-étape de calcul d'un coefficient de pondération pour chacun desdits genres associés audit état émotionnel détecté comprend :
- pour ledit état émotionnel détecté, la détermination d'une valeur maximale parmi lesdits compteurs associés à chacun desdits genres ;
- pour chaque genre associé audit état émotionnel détecté, la division de ladite valeur dudit compteur correspondant par ladite valeur maximale déterminée.

Selon un autre aspect particulier de cet autre mode particulier, ladite étape de réalisation d'un tri desdits programmes dudit catalogue tenant au moins compte dudit état émotionnel détecté et desdits genres associés audit état émotionnel détecté comprend une sous-étape de
- hiérarchisation des programmes d'une liste de programmes proposés, dans laquelle chaque programme présente un score prédéterminé, par ordre décroissant dudit compteur associé audit genre et audit état émotionnel détecté ; puis
- hiérarchisation desdits programmes de ladite liste de programmes ayant le même genre par ordre décroissant du score prédéterminé.

Selon un aspect particulier de l'invention, le procédé comprend une étape de mise à jour de ladite table de priorité ou de ladite table de comptage, lorsque ledit au moins un utilisateur lit pendant au moins une durée prédéterminée un desdits programmes recommandés, par incrémentation d'une valeur dudit compteur associé audit programme lu par ledit au moins un utilisateur.

Selon un autre aspect particulier de l'invention, chaque genre comprend au moins un sous-genre et ladite table de priorité et ladite table de comptage comprennent un compteur de lectures pour chaque genre et un compteur de lectures pour chaque sous-genre. L'étape de mise à jour de ladite table de priorité et de ladite table de comptage comprenant les sous-étapes de, lorsque ledit au moins un utilisateur lit un desdits programmes recommandés :
- incrémentation desdits compteurs du genre et du sous-genre du programme sélectionné par ledit au moins un utilisateur correspondant audit état émotionnel détecté d'une première valeur ;
- incrémentation des compteurs des autres sous-genres associés au genre du programme sélectionné par ledit au moins un utilisateur correspondant audit état émotionnel détecté d'une deuxième valeur.

Selon un aspect particulier, lorsque ledit décodeur audiovisuel détecte au moins deux utilisateurs, le procédé comprend :
- une étape de détermination de l'utilisateur principal sur la base d'au moins un critère prédéterminé, ladite sous-étape d'utilisation d'une table de priorité ou d'une table de comptage s'effectuant à partir de ladite table de priorité ou de ladite table de comptage associée audit utilisateur principal déterminé ; ou
- une étape de combinaison desdites tables de priorité ou de comptage de chacun desdits utilisateurs détectés, ladite sous-étape d'utilisation d'une table de priorité ou d'une table de comptage s'effectuant à partir de la table de priorité ou de la table de comptage issue de cette combinaison.

Selon un aspect particulier de l'invention, le procédé comprend une étape de déclenchement dudit procédé :
- à la mise en route dudit décodeur audiovisuel ; ou
- à la détection d'au moins un utilisateur par ledit décodeur audiovisuel ; ou
- à la sélection/recherche d'un programme par ledit au moins un utilisateur.

L'invention propose également un décodeur audiovisuel pour la mise en oeuvre du procédé de recommandation d'un programme ou d'une liste de programmes audiovisuels parmi un catalogue de programmes à au moins un utilisateur tel que décrit précédemment, lesdits programmes dudit catalogue étant catégorisés en genres. Ledit décodeur audiovisuel étant destiné à être connecté à une base de données distante, un moyen d'affichage et un dispositif de commande utilisable par un utilisateur pour commander ledit décodeur audiovisuel, ledit décodeur comprenant une circuiterie électronique adaptée et configurée pour :
- associer lesdits genres à des états émotionnels ;
- détecter un état émotionnel dudit au moins un utilisateur ;
- réaliser un tri desdits programmes dudit catalogue tenant au moins compte dudit état émotionnel détecté et desdits genres associés audit état émotionnel détecté
- lesdits moyens d'affichage étant destinés à afficher une liste d'au moins un programme audiovisuel tenant compte dudit tri desdits programmes dudit catalogue.

Il est également proposé ici un programme d'ordinateur, qui peut être stocké sur un support et/ou téléchargé d'un réseau de communication, afin d'être lu par un processeur. Ce programme d'ordinateur comprend des instructions pour implémenter le procédé mentionné ci-dessus dans l'un quelconque de leurs modes de réalisation, lorsque ledit programme est exécuté par le processeur. L'invention concerne également un support de stockage d'informations stockant un tel programme d'ordinateur.

### BREVE DESCRIPTION DES DESSINS

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
[Fig. 1] illustre schématiquement un système de télécommunication dans lequel la présente invention est implémentée ;
[Fig. 2] illustre schématiquement un exemple d'architecture matérielle d'un décodeur audiovisuel de l'invention ;
[Fig. 3] illustre schématiquement un procédé de recommandation selon l'invention ;
[Fig. 4] illustre schématiquement une étape d'association des genres à des états émotionnels du procédé de la Fig. 3, selon un premier mode de réalisation ;
[Fig. 5] représente un exemple de table de priorité mise en oeuvre dans le procédé de la Fig. 3, la table de priorité étant dans un premier état ;
[Fig. 6] illustre schématiquement une étape de réalisation d'un tri des programmes d'un catalogue tenant au moins compte d'un état émotionnel détecté et des genres associés à l'état émotionnel détecté du procédé de la Fig. 3, selon le premier mode de réalisation ;
[Fig. 7] représente un exemple de table de priorité lors de la mise en oeuvre dans le procédé de la Fig. 3, la table de priorité étant dans un deuxième état ;
[Fig. 8] représente un exemple de table de priorité lors de la mise en oeuvre dans le procédé de la Fig. 3, la table de priorité étant dans un état initial ;
[Fig. 9] illustre schématiquement une étape d'affichage d'une liste d'au moins un programme audiovisuel tenant compte du tri des programmes du catalogue du procédé de la Fig. 3 ;
[Fig. 10] illustre schématiquement une méthode de remplissage de la table de priorité par apprentissage, selon le premier mode de réalisation ;
[Fig. 11] illustre schématiquement une étape d'association des genres à des états émotionnels du procédé de la Fig. 3, selon un deuxième mode de réalisation ;
[Fig. 12] représente un exemple de table de comptage mise en oeuvre dans le procédé de la Fig. 3, la table de comptage étant dans un état initial ;
[Fig. 13] illustre schématiquement une étape de réalisation d'un tri des programmes d'un catalogue tenant au moins compte d'un état émotionnel détecté et des genres associés à l'état émotionnel détecté du procédé de la Fig. 3, selon le deuxième mode de réalisation ; [Fig. 14] représente un exemple d'une liste de programmes proposée par l'opérateur, chaque programme comprenant un score prédéterminé ;
[Fig. 15] représente un exemple de table de comptage mise en oeuvre dans le procédé de la Fig. 3, dans laquelle les compteurs ont été incrémentés suite à la mise en oeuvre du procédé de la Fig. 3 ;
[Fig. 16] illustre un exemple de tableau de calcul d'un score pondéré ;
[Fig. 17] illustre un exemple de tableau de pondération d'une liste de programmes proposée par l'opérateur ;
[Fig. 18] illustre un exemple d'une liste de programmes hiérarchisés par la mise en oeuvre du procédé de la Fig. 3, selon le deuxième mode de réalisation ;
[Fig. 19] illustre schématiquement une étape de mise à jour de la table de comptage, selon le deuxième mode de réalisation ;
[Fig. 20] illustre schématiquement une variante d'une étape de réalisation d'un tri des programmes d'un catalogue tenant au moins compte d'un état émotionnel détecté et des genres associés à l'état émotionnel détecté du procédé de la Fig. 3, selon le deuxième mode de réalisation ;
[Fig. 21] illustre schématiquement une variante d'une étape de mise à jour de la table de priorité ou de la table de comptage, compatible avec les premier et deuxième modes de réalisation ; et
[Fig. 22] illustre schématiquement une étape de détection du nombre d'utilisateurs du procédé de la Fig. 3.

### EXPOSE DETAILLE DE MODES DE REALISATION

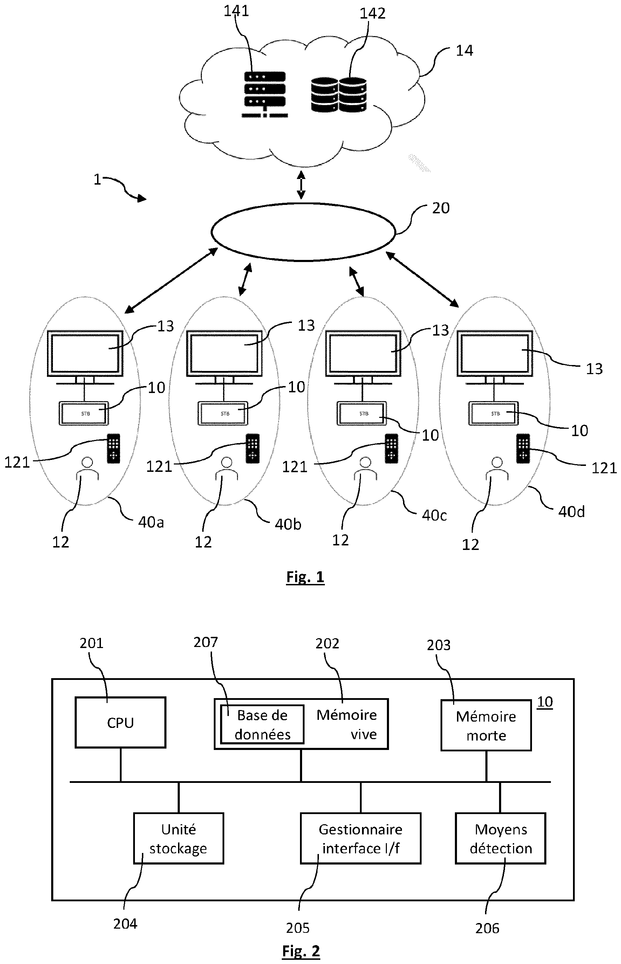

La Fig. 1 illustre schématiquement un système 1 de télécommunication dans lequel la présente invention est implémentée.

Dans la Fig. 1, une source de données externes 14 comprenant un serveur 141 et une base de données distantes 142 est reliée par l'intermédiaire d'un réseau étendu 20 (Wide Area Network en anglais) à des réseaux locaux 40 (LAN en anglais). Le réseau étendu 20 est par exemple un réseau de type internet. La base de données distantes 142 est par exemple configurée pour stocker les informations relatives aux programmes audiovisuels.

Par exemple les réseaux locaux 40 sont des réseaux domestiques filaire et/ou sans fils. Seulement quatre réseaux domestiques 40a - 40d sont représentés en Fig. 1 par souci de simplification. Dans un exemple particulier, les réseaux locaux 40a - 40d sont des réseaux sans fils de type Wi-Fi.

Dans l'exemple de la Fig. 1, chaque réseau local 40a - 40d comprend un décodeur audiovisuel 10 permettant de mettre en oeuvre un procédé de recommandation d'au moins une liste de programmes. Le décodeur 10 est connecté à au moins un moyen d'affichage 13 (par un lien HDMI (High Définition Multimedia Interface en anglais), SCART (Syndicat des Constructeurs d'Appareils Radiorécepteurs et Téléviseurs), ou tout autre type de lien adapté) et à un dispositif de commande 121 (de préférence via une connexion sans fil) utilisé par au moins un utilisateur 12 pour commander le décodeur audiovisuel 10. Selon une approche, l'utilisateur peut interagir avec le décodeur audiovisuel 10 via un microphone intégré au décodeur audiovisuel 10. Dans l'exemple illustré, un seul utilisateur et un seul moyen d'affichage sont représentés.

La Fig. 2 illustre schématiquement un exemple d'architecture matérielle permettant d'implémenter, sous forme de circuiterie électronique, le décodeur audiovisuel selon l'invention.

Selon l'exemple d'architecture matérielle représenté à la Fig. 2, le décodeur 10 comprend, reliés par un bus de communication 200 : un processeur ou CPU (« Central Processing Unit » en anglais) 201 ; une mémoire vive RAM (« Random Access Memory » en anglais) 202 ; une mémoire morte 203, par exemple de type ROM (« Read Only Memory » en anglais) ou EEPROM (« Electrically-Erasable Programmable ROM » en anglais), telle qu'une mémoire Flash ; une unité de stockage 204, telle qu'un disque dur HDD (« Hard Disk Drive » en anglais) ou un lecteur de support de stockage, tel qu'un lecteur de cartes SD (« Secure Digital » en anglais)); au moins un gestionnaire d'interfaces Vf 205. Le gestionnaire d'interface I/f 205 permet au décodeur audiovisuel 10 de communiquer par l'intermédiaire du réseau étendu 20 et d'interagir avec le dispositif de commande 121.

Le processeur 201 est capable d'exécuter des instructions chargées dans la RAM 202 à partir de la ROM 203, d'une mémoire externe (non représentée), d'un support de stockage (tel qu'une carte SD), ou d'un réseau de communication. Lorsque le décodeur audiovisuel 10 est mis sous tension, le processeur 201 est capable de lire de la RAM 202 des instructions et de les exécuter. Ces instructions forment un programme d'ordinateur causant la mise en oeuvre, par le processeur 201, de tout ou partie du procédé décrit ici. L'unité de stockage 204, ou la mémoire vive 202, est apte à stocker une base de données locale 207 stockant les tables de priorité et/ou de comptage décrites plus en détails dans la suite de cette description.

Le décodeur 10 comprend en outre des moyens de détection 206 de l'émotion d'un utilisateur. Pour ce faire, les moyens de détection 206 comprennent des moyens de capture vidéo et/ou audio (par exemple au moins une caméra et/ou un microphone) de l'utilisateur ainsi qu'un module d'association configuré pour associer les images et/ou l'audio captés avec un état émotionnel courant de l'utilisateur. Optionnellement, les moyens de détection 206 peuvent mettre en oeuvre un dispositif connecté du type IoT (pour « Internet des Objets) basé sur des biomarqueurs.

Tout ou partie du procédé décrit ici peut ainsi être implémenté sous forme logicielle par exécution d'un ensemble d'instructions par une machine programmable, par exemple un processeur de type DSP (« Digital Signal Processor » en anglais) ou un microcontrôleur, ou être implémenté sous forme matérielle par une machine ou un composant électronique (« chip » en anglais) dédié ou un ensemble de composants électroniques (« chipset » en anglais) dédié, par exemple un composant FPGA (« Field Programmable Gate Array » en anglais) ou ASIC (« Application Spécifie Integrated Circuit » en anglais). D'une manière générale, le décodeur audiovisuel 10 comporte de la circuiterie électronique adaptée et configurée pour implémenter le procédé décrit ici.

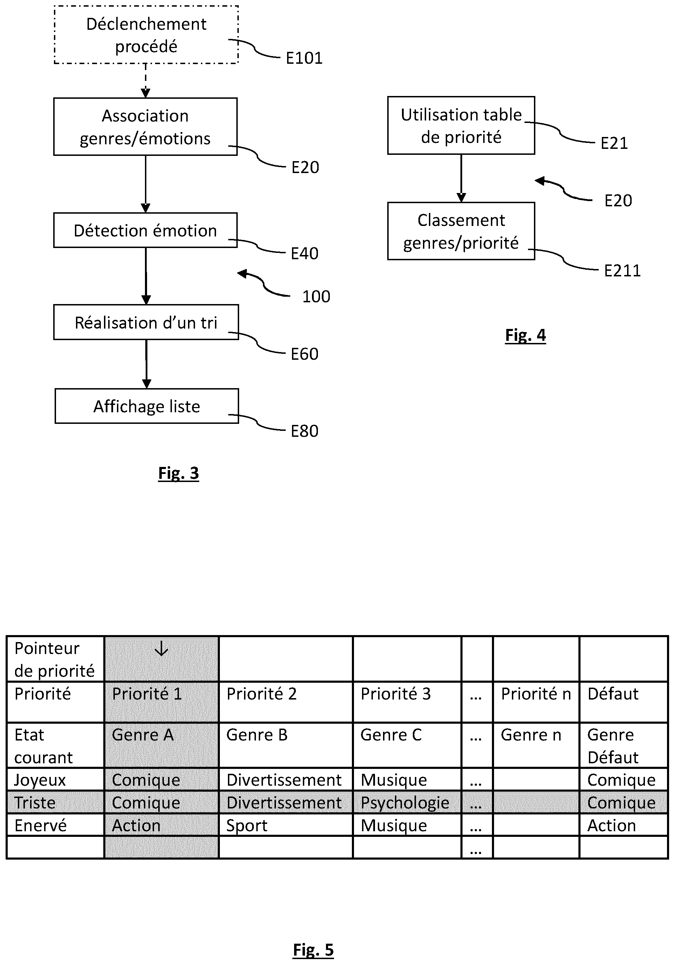

Le procédé décrit ci-après en relation avec les Figs. 3 à 22 peut être implémenté sous forme logicielle par exécution d'un ensemble d'instructions par une machine programmable, par exemple un DSP (« Digital Signal Processor » en anglais) ou un microcontrôleur, ou être implémenté sous forme matérielle par une machine ou un composant dédié, par exemple un FPGA (« Field-Programmable Gate Array » en anglais) ou un ASIC (« Application-Specific Integrated Circuit » en anglais). En général, le décodeur audiovisuel 10 comprend de la circuiterie électronique configurée pour mettre en oeuvre le procédé décrit en relation avec les Figs. 3 à 22.

Il est à remarquer ici que la Fig. 2 représente une architecture matérielle d'un unique décodeur audiovisuel 10. Une partie des différents éléments constitutifs du décodeur audiovisuel 10 peut être répartie dans différents dispositifs informatiques compris dans un nuage informatique.

La Fig. 3 illustre schématiquement un procédé 100 de recommandation d'une liste d'au moins un programme audiovisuel parmi un catalogue de programmes audiovisuels selon la présente invention. Le procédé 100 de recommandation est destiné à au moins un utilisateur 12 orienté vers le décodeur audiovisuel 10 et vers les moyens d'affichage 13. Il est à noter que les programmes du catalogue de programmes sont catégorisés en genres et selon une autre approche, en genres et en sous-genres. Les informations relatives à la catégorie de chaque programme peuvent être récupérées par le décodeur audiovisuel 10 selon des méthodes connues. Par exemple, la diffusion d'un programme sur des réseaux satellite, câble ou de télévision terrestre passe par la norme DVB (Digital Video Broadcasting en anglais) qui prévoit le signalement de métadonnées liées à un programme via la table EIT (« Event Information Table » en anglais). Une table EIT peut notamment indiquer le genre du programme via la description du contenu, comme détaillé dans le document ETSI EN 300 468. Le décodeur audiovisuel 10 de l'invention est donc apte à obtenir les informations relatives au genre des programmes par un moyen adapté (du type liaison IP (Protocole Internet), DSL (Digital Subscriber Line en anglais), câble, satellite, ...), depuis la source de données externe 14, qui peut être une entrée Satellite, Câble, TNT (Télévision Numérique Terrestre), IP ou toute autre source de flux audiovisuel.

Selon une autre approche, les informations relatives aux programmes peuvent être récupérées par le décodeur audiovisuel 10 via un fichier texte de description (JSON par exemple (JavaScript Object Notation en anglais)) téléchargé depuis un serveur Internet. De manière générale, les genres de programme possibles utilisés selon l'invention sont ceux donnés par le fournisseur de contenus (DVB, OTT (Over-The-Top en anglais), etc.).

Par exemple, les programmes peuvent être catégorisés selon les genres suivants : Film/Drame, Actualités, Spectacle/jeu télévisé, Sports, Programme pour enfants/jeunes, Musique/Ballet/Danse, Arts/Culture (sans musique), Questions sociales / politiques / économiques, Éducation/science/sujets factuels, Loisirs, etc.

De manière optionnelle, les programmes peuvent en outre être catégorisés en sous-genres, comme par exemple pour le genre Film/drame : Général, Détective/thriller, Aventure/western/guerre, Science-fiction/fantastique/horreur, Comédie, Feuilleton/mélodrame/folklorique, Romance, Film/drame sérieux / classique / religieux / historique, Film/drame pour adultes.

On comprend bien évidemment que l'invention peut utiliser tout ou partie des genres listés ci-dessus. L'invention peut également utiliser d'autres genres non listés ci-dessus. De plus, l'invention peut utiliser les genres principaux seuls ou en combinaison avec leurs sous-genres.

Par ailleurs, le décodeur audiovisuel 10 dispose d'une base de données 207 regroupant un certain nombre d'états émotionnels déjà détectés ou qu'il est capable de détecter (comme décrit ci-après) par les moyens de détection 206. Ces états émotionnels sont par exemple compris parmi : la colère, le dégoût, la peur, la joie, la tristesse, la surprise, la neutralité, etc. On comprend bien évidemment que l'invention peut utiliser tout ou partie des états émotionnels listés ci-dessus. L'invention peut également utiliser d'autres états émotionnels non listés ci-dessus.

Selon l'invention, le procédé 100 de recommandation comprend les étapes, mises en oeuvre par le décodeur audiovisuel 10, de :
- association E20 des genres à des états émotionnels ;
- détection E40 d'un état émotionnel courant de l'utilisateur ;
- réalisation d'un tri E60 desdits programmes dudit catalogue tenant au moins compte dudit état émotionnel détecté et desdits genres associés audit état émotionnel détecté ;
- affichage E80 d'une liste d'au moins un programme audiovisuel tenant compte dudit tri desdits programmes dudit catalogue.

Afin de faciliter la compréhension du procédé selon le premier mode de réalisation, ce dernier est décrit en ne tenant compte que des genres des programmes (et pas des sous-genres). On comprend aisément que le procédé de l'invention pourrait tenir compte, en complément ou à la place des genres, des sous-genres des programmes sans s'écarter du principe de fonctionnement de ce mode de réalisation. Un exemple de réalisation tenant compte des sous-genres est notamment décrit dans la suite de la description.

Selon un premier mode de réalisation, l'étape d'association E20 des genres à des états émotionnels comprend, comme illustré sur la Fig. 4, une sous-étape d'utilisation E21 d'une table de priorité. Plus précisément, la table de priorité comprend une liste d'états émotionnels courants auxquels sont associées des propositions de genres de programmes. La table de priorité permet de classer E211 selon un ordre de priorité chaque genre proposé pour chaque état émotionnel. Ce classement E211 des genres associés à chaque état émotionnel tient au moins compte d'un compteur de lectures, par l'utilisateur, de chaque genre associé à chaque état émotionnel. En d'autres termes, pour chaque état émotionnel, on classe les genres par ordre de priorité, c'est-à-dire que pour un état émotionnel donné, les genres sont classés par ordre de genres les plus lus par l'utilisateur lorsque l'utilisateur est dans l'état émotionnel donné. Les compteurs de lectures comptabilisent localement le nombre de lectures par l'utilisateur, par exemple au sein du foyer.

Par exemple, et comme illustré sur de la Fig. 5 qui représente une table de priorité selon le premier mode de réalisation, pour l'état émotionnel correspondant à la tristesse, le genre classé en premier selon l'ordre de priorité est le genre comique, puis le genre divertissement puis le genre psychologie. Cela signifie que lorsque l'utilisateur est dans l'état émotionnel correspondant à la tristesse, il a regardé davantage de programmes du genre comique, puis du genre divertissement puis du genre psychologie.

Comme précité, le classement des genres par état émotionnel tient notamment compte de d'un compteur de lectures associées à chaque genre pour chaque état émotionnel. D'autres critères peuvent toutefois être pris en compte pour classer les genres associés à chaque état émotionnel. De plus, il est aussi envisageable de prédéfinir l'ordre de préférence des genres à proposer pour chaque état émotionnel. Cet ordre prédéfini pourrait par exemple être configuré par l'utilisateur lors de la configuration du décodeur audiovisuel 10 ou par l'opérateur fournissant le décodeur audiovisuel 10.

On note que dans la table de priorité représentée sur la Fig. 5, seuls trois genres sont détaillés mais n genres peuvent être associés à chaque état émotionnel. On comprend bien évidemment qu'un nombre plus élevé ou moins élevé que trois genres pourrait être associé à chaque état émotionnel. Le nombre de genres associé à chaque état émotionnel pourrait également être limité, par exemple à 20 genres par état émotionnel, de sorte à limiter la taille de la table de priorité et optimiser l'espace de stockage, notamment. Optionnellement le nombre de genres pris en compte dans la table de priorité (c'est-à-dire le nombre de priorités) pourrait correspondre au nombre total de genres proposés par la norme de diffusion des contenus audiovisuels. Optionnellement le nombre de genres pris en compte dans la table de priorité pourrait être étendu, par exemple par le diffuseur ou par le décodeur audiovisuel 10 lors de la détection de l'ajout d'un nouveau genre. Cet ajout peut par exemple se faire en fin de table avec l'ajout d'une nouvelle colonne dans la table de priorité.

De plus, il est prévu, pour chaque état émotionnel, un genre par défaut (et éventuellement un sous-genre par défaut) qui est proposé à l'utilisateur lors de l'initialisation de la table de priorité ou lorsque l'utilisateur ne sélectionne aucun des genres proposés, comme décrit ci-après.

La table de priorité peut être embarquée dans le décodeur audiovisuel 10 ou téléchargée depuis un réseau extérieur. Par exemple, une implémentation possible de la table de priorité peut être faite en SQL (Structured Query Language en anglais) ou bien encore cela peut être un fichier du type JSON.

L'étape E40 de détection d'un état émotionnel courant de l'utilisateur 12 peut par exemple mettre en oeuvre une technique de détection connue qui consiste à utiliser des réseaux de neurones entraînés pour détecter les émotions d'un individu à partir d'images. Cette technique peut par exemple mettre en oeuvre, pour l'extraction des caractéristiques du visage de l'individu à partir de l'image : des algorithmes du type filtres de Gabor, histogramme de gradients orientés (HOG - Histogram of Oriented Gradients en anglais) ou un motif binaire local (LBP - Local Binary Pattern en anglais). Par la classification, cette technique peut par exemple mettre en oeuvre une machine à vecteur de support (SVM - Support Vector Machine en anglais), une forêt aléatoire (RF - Random Forest en anglais) ou un algorithme des plus proches voisins (kNN - Nearest Neighbor Algorithm en anglais). D'autres techniques permettent de détecter l'état émotionnel d'un individu à partir de sa voix, comme décrit dans les documents US11341986 ou TW201606760A.

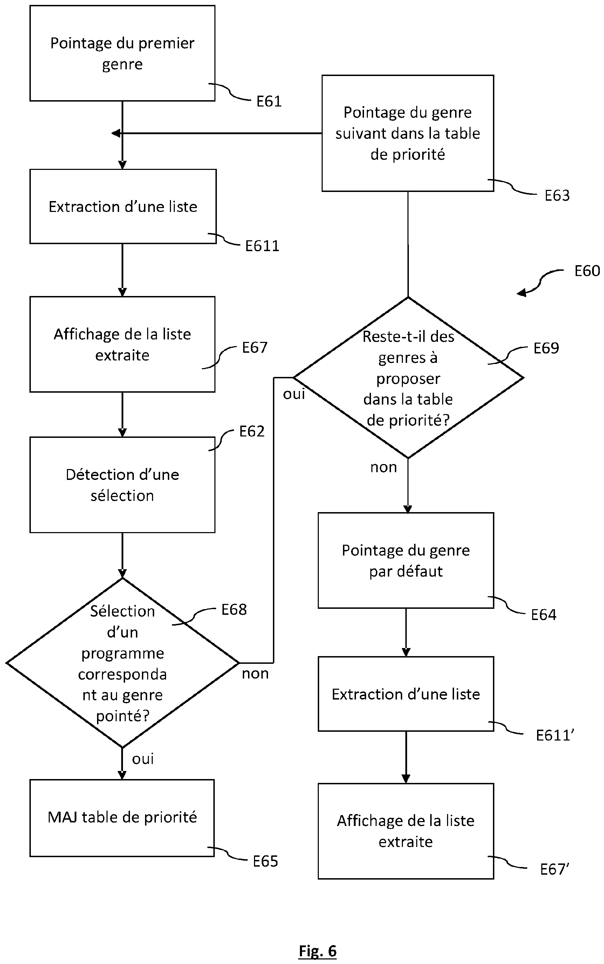

Selon le premier mode de réalisation, l'étape de réalisation d'un tri E60 des programmes du catalogue tenant au moins compte de l'état émotionnel détecté et des genres (et/ou sous-genres lorsque le procédé tient compte des sous-genres) associés à l'état état émotionnel détecté permet d'extraire un programme ou une liste de programmes à proposer/recommander à l'utilisateur en fonction de son état émotionnel courant détecté. Pour ce faire, le décodeur audiovisuel 10 se réfère à la table de priorité de la Fig. 5. Le décodeur audiovisuel 10 implémente l'étape de réalisation d'un tri E60 qui comprend les sous-étapes de (illustrées sur la Fig. 6) :
- pointage E61, dans ladite table de priorité, du genre classé en premier selon l'ordre de priorité pour l'état émotionnel détecté, et
- détection E62 d'une sélection par l'utilisateur et, si l'utilisateur ne sélectionne pas un programme correspondant audit premier genre pointé, pointage E63 du genre suivant dans la table de priorité jusqu'à ce que l'utilisateur sélectionne un programme dudit genre pointé ou jusqu'à ce que ledit genre pointé soit ledit genre par défaut E64.

Plus précisément, et en se référant à la table de priorité illustrée sur la Fig. 5, lorsqu'un état émotionnel est détecté, le pointeur est placé sur le premier genre selon l'ordre de priorité de la table (comme illustré sur la Fig. 5). Si l'on continue avec l'exemple de l'état émotionnel correspondant à la tristesse, le premier genre selon l'ordre de priorité est le genre comique. C'est donc le genre comique qui est pointé (voir la flèche sur la Fig. 5) au moment de la sous-étape E61.

Une sous-étape E611 d'extraction d'une liste d'au moins un programme correspondant au genre pointé parmi les programmes du catalogue est donc implémentée par le décodeur audiovisuel 10. Ensuite, une sous-étape E67 d'affichage de la liste extraite comportant au moins un programme audiovisuel correspondant au genre sélectionné, c'est-à-dire au genre pointé dans la table, est donc implémentée. La sous-étape d'affichage E67correspond à l'étape d'affichage E80 telle que décrite ci-après.

A la sous-étape E62, le décodeur audiovisuel 10 détecte si l'utilisateur sélectionne un programme parmi les programmes de la liste extraite correspondant au genre pointé dans la table de priorité.

A la sous-étape E68, le décodeur audiovisuel 10 vérifie si l'utilisateur sélectionne un programme parmi cette liste extraite de programmes correspondant au genre pointé.

Si l'utilisateur sélectionne un programme parmi cette liste extraite de programmes correspondant au genre pointé dans la table de priorité, le décodeur passe à la sous-étape E65.

Si l'utilisateur ne sélectionne pas un programme parmi cette liste extraite de programmes correspondant au genre pointé dans la table de priorité, et s'il reste des genres à proposer dans la table de priorité, le décodeur audiovisuel 10 vérifie, à la sous-étape E69, s'il reste des genres à proposer dans la table de priorité. Si oui, le décodeur audiovisuel 10 passe à la sous-étape E63 dans laquelle le pointeur est décalé d'une colonne dans la table de priorité et une sous-étape E611 d'extraction d'une nouvelle liste d'au moins un programme correspondant au genre pointé puis une sous-étape E67 d'affichage d'une liste d'au moins un programme audiovisuel correspondant au genre sélectionné, c'est-à-dire au genre pointé dans la table, sont donc implémentées. S'il ne reste aucun genre à proposer dans la table de priorité, le décodeur audiovisuel 10 pointe E64 le genre par défaut et une sous-étape E611' d'extraction d'une nouvelle liste d'au moins un programme correspondant au genre par défaut puis une sous-étape E67' d'affichage d'une liste d'au moins un programme audiovisuel correspondant au genre par défaut sont implémentées.

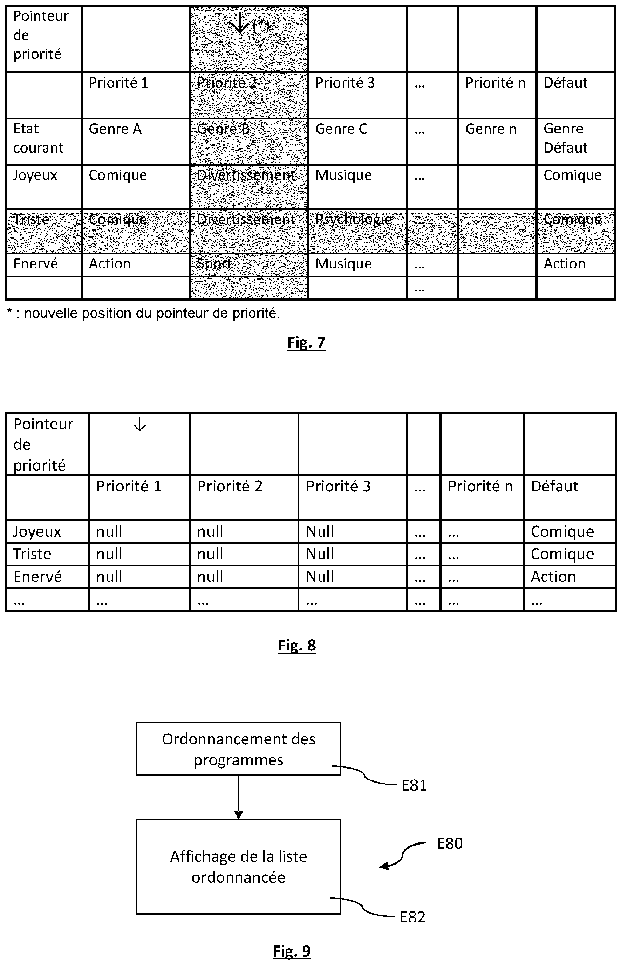

Dans le présent exemple relatif à l'état émotionnel « triste », si l'utilisateur ne sélectionne pas un programme correspondant au genre « comique », alors le pointeur est décalé sur le genre « divertissement », comme illustré sur la Fig. 7. Une liste d'au moins un programme audiovisuel correspondant au genre divertissement est alors extraite puis affichée. Si l'utilisateur ne sélectionne toujours pas un programme dans la liste extraite des programmes correspondant au genre divertissement, alors le décodeur audiovisuel 10 passe à la sous-étape E69 et s'il reste des genres à proposer dans la table de priorité, le décodeur audiovisuel 10 passe à la sous-étape E63 de sorte que le pointeur soit décalé au genre suivant dans la table de priorité (genre psychologie), et ainsi de suite tant qu'il reste des genres à proposer dans la table de priorité. Ainsi, le décodeur audiovisuel 10 vérifie à la sous-étape E69 qu'il reste des genres à proposer dans la table de priorité. Dans l'affirmative, le décodeur passe à l'étape E63 tandis que dans la négative, le décodeur audiovisuel 10 passe à l'étape E64.

De cette manière, le pointeur est décalé jusqu'à ce que le pointeur atteigne le genre par défaut, qui est classé en dernier dans la table de priorité pour chaque état émotionnel (ici le genre par défaut associé à l'état émotionnel tristesse est le genre comique). Le genre par défaut est défini au préalable. Par exemple, il peut être défini de manière automatique par le décodeur audiovisuel 10 ou manuellement par l'utilisateur lors de la configuration du décodeur audiovisuel 10.

En d'autres termes, le pointeur parcourt la table de priorité pour proposer successivement les genres classés dans la table de priorité correspondant à l'état émotionnel détecté. Si aucun programme correspondant à ces genres n'est sélectionné par l'utilisateur, un genre par défaut est sélectionné et une liste d'au moins un programme associé à ce genre par défaut est extraite et proposée.

Dans le cas où l'utilisateur sélectionne un programme parmi la liste extraite de programmes proposée correspondant à un genre pointé dans la table de priorité, alors le décodeur audiovisuel 10 met à jour la table de priorité (MAJ sous-étape E65) en incrémentant la valeur du compteur du nombre de lectures pour le genre correspondant au programme sélectionné pour l'état émotionnel détecté et les différents genres appartenant à l'état émotionnel détecté sont reclassé de manière que :
- la priorité 1 contienne le genre ayant le plus de lectures,
- la priorité 2 contienne le second genre ayant le plus de lectures suivant le genre ayant la priorité 1 ; etc.

L'étape E80 d'affichage d'une liste d'au moins un programme tenant compte dudit tri desdits programmes dudit catalogue comprend, dans cet exemple, les sous-étapes de (illustrées sur la Fig. 9) :
- ordonnancement E81 de ladite liste d'au moins un programme à afficher, l'ordonnancement tenant au moins en partie compte d'un critère de priorité du au moins un programme ; et
- affichage E82 de la liste d'au moins un programme en fonction de l' ordonnancement.

Plus précisément, lorsque l'étape de réalisation d'un tri E60 des programmes du catalogue tenant au moins compte de l'état émotionnel détecté et des genres associés à l'état émotionnel détecté est effectuée, une liste d'au moins un programme correspondant au genre pointé est extraite. C'est cette liste extraite qu'il est nécessaire d'afficher E80 à l'utilisateur. L'ordonnancement des programmes de la liste extraite tient au moins compte d'un critère de priorité associé à chaque programme. Par exemple, le critère de priorité peut tenir compte de la popularité du programme auprès d'autres utilisateurs, de sa date de sortie ou de mise à disposition, ou de son apport commercial/marketing (par exemple, les publicités diffusées dans certains programmes se vendent plus cher que dans d'autres, le producteur d'un programme peut payer pour que celui-ci ait une visibilité accrue, etc.). Une combinaison de ces données peut être utilisée pour déterminer le critère de priorité de chaque programme. D'autres données peuvent évidemment être utilisées dans la détermination de ce critère de priorité. De préférence, le critère de priorité est préalablement reçu par le décodeur audiovisuel 10, par exemple lors de la réception des informations relatives aux programmes audiovisuels

Dans cet exemple, les programmes de cette liste extraite sont ordonnancés en fonction de leur popularité auprès des autres utilisateurs. Ici, les programmes proposés sont donc ordonnancés du programme le plus populaire au programme le moins populaire auprès des autres utilisateurs. Par exemple, la popularité d'un programme peut être déterminée en tenant compte du nombre de lectures du programme, de sa notation par les autres utilisateurs, de sa notation par les plateformes, de sa notation par les médias ou de tout autre critère. Une combinaison de ces critères peut également être utilisée pour déterminer la popularité du programme.

Lorsque les programmes de la liste sont ordonnancés, le décodeur audiovisuel 10 affiche, via les moyens d'affichage 13, la liste ordonnancée à l'utilisateur pour faciliter sa sélection. Un exemple de liste de programmes ordonnancés peut consister en la présentation du Top 10 des films comiques les plus regardés (pour l'état émotionnel « triste » en priorité 1) ou du Top 10 des émissions de divertissement les mieux notés (pour l'état émotionnel « triste » en priorité 2), etc.

La Fig. 8 représente une table de priorité vide, telle qu'elle peut être lors de l'initialisation du décodeur audiovisuel 10. À l'initialisation du décodeur audiovisuel 10, la colonne défaut proposant un genre par défaut à chaque état émotionnel peut toutefois être automatiquement renseignée.

Le remplissage de la table de priorité à son initialisation peut être effectué en se basant sur un panel d'utilisateurs et en calquant le contenu de la table de priorité sur celui du panel d'utilisateurs.

Une autre solution peut consister à remplir la table de priorité par apprentissage, c'est-à-dire au fur et à mesure que l'utilisateur sélectionne un programme.

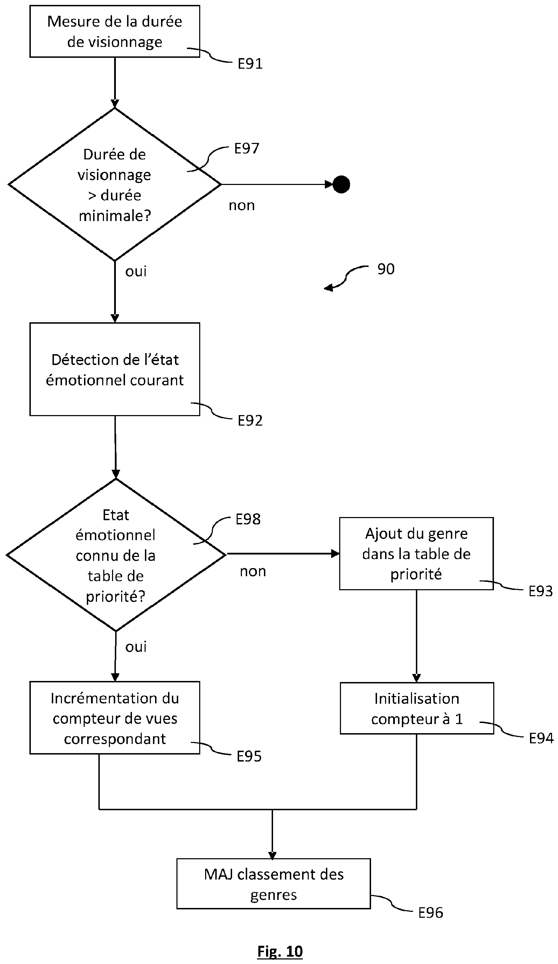

La Fig. 10 illustre un exemple de méthode de remplissage de la table de priorité par apprentissage. Cette méthode de remplissage 90 comprend les étapes de :
- mesure par le décodeur audiovisuel 10 à l'étape E91 de la durée de visionnage d'un programme par l'utilisateur ;
- vérification par le décodeur audiovisuel 10 à l'étape E97 si la durée de visionnage mesurée est supérieure à une durée minimale prédéterminée (par exemple de dix minutes). Dans la négative, le décodeur audiovisuel 10 interrompt le présent algorithme. Dans l'affirmative, le décodeur audiovisuel 10 passe à l'étape E92 de détection de l'état émotionnel courant de l'utilisateur ;
- A l'étape E98, le décodeur audiovisuel 10 vérifie si l'état émotionnel détecté ne comporte pas le genre du programme visionné par l'utilisateur dans la table de priorité. Dans l'affirmative, le décodeur audiovisuel 10 passe à l'étape E93. Dans la négative, le décodeur audiovisuel 10 passe à l'étape E95. A l'étape E93, le décodeur audiovisuel 10 ajoute le genre du programme visionné par l'utilisateur pour l'état émotionnel détecté dans la table de priorité et initialise à l'étape E94 le compteur de lectures correspondant à 1 ;
- si l'état émotionnel détecté contient déjà le genre du programme visionné par l'utilisateur dans la table de priorité, le décodeur audiovisuel 10 incrémente E95 d'une unité de la valeur du compteur de lectures du genre correspondant à l'état émotionnel détecté ;
- le décodeur audiovisuel 10 met à jour à l'étape E96 le classement des genres par ordre de priorité pour l'état émotionnel détecté.

Cette méthode peut être mise en oeuvre en parallèle du procédé de recommandation 100 décrit précédemment.

Selon un deuxième mode de réalisation, le procédé de recommandation 100 fait intervenir l'état émotionnel courant de l'utilisateur détecté pour pondérer un score prédéterminé associé à un programme de sorte à effectuer une recommandation de programmes à l'utilisateur qui tienne compte de son état émotionnel courant.

Afin de faciliter la compréhension du procédé selon le deuxième mode de réalisation, ce dernier est décrit en ne tenant compte que des genres des programmes (et pas des sous-genres). On comprend aisément que le procédé de l'invention pourrait tenir compte, en complément ou à la place des genres, des sous-genres des programmes sans s'écarter du principe de fonctionnement de ce mode de réalisation. Un exemple de réalisation tenant compte des sous-genres est notamment décrit dans la suite de la description.

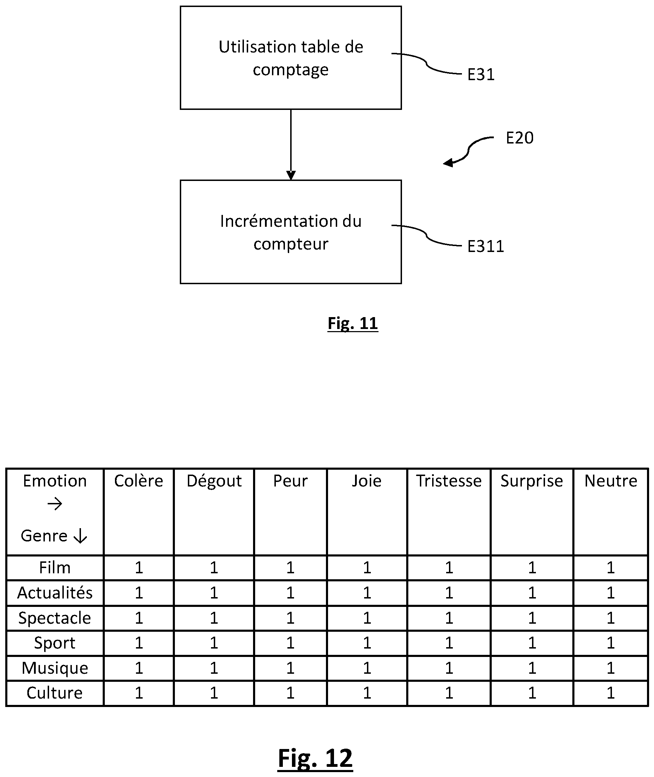

Pour ce faire, à l'étape d'association E20 des genres (et/ou éventuellement des sous-genres) à des états émotionnels, le décodeur audiovisuel 10 implémente, comme illustré sur la Fig. 11, une sous-étape d'utilisation E31 d'une table de comptage comprenant un compteur de lectures par l'utilisateur pour chaque genre associé à un état émotionnel. Plus précisément, la table de comptage comprend une liste d'états émotionnels courants auxquels sont associées des propositions de genres de programmes. La table de comptage permet au décodeur audiovisuel 10 de compter E311, en incrémentant d'une valeur un compteur de lectures associé à chaque genre pour chaque état émotionnel, le nombre de lectures d'un programme de chaque genre associé à chaque état émotionnel lorsque l'utilisateur sélectionne un programme proposé par le procédé de l'invention. En d'autres termes, à chaque fois qu'un utilisateur sélectionne et lit un programme qui lui est proposé par le procédé de l'invention, le décodeur audiovisuel 10 incrémente d'une valeur le compteur correspondant au genre associé à l'état émotionnel détecté. De cette manière, la table de comptage permet de compter et stocker le nombre de lectures de chaque genre associé à chaque état émotionnel.

Il est également envisageable de compter les lectures qui sont effectuées hors du procédé de l'invention. Par exemple, il est envisageable de compter les lectures effectuées par une sélection spontanée de l'utilisateur ou lorsque l'utilisateur zappe d'un programme à un autre, notamment. Afin d'optimiser la pertinence des recommandations, la table de comptage peut également être remplie par apprentissage, d'une manière sensiblement similaire à la technique décrite ci-dessus en relation avec la Fig. 10.

La Fig. 12 représente une table de comptage lors de l'initialisation du décodeur audiovisuel 10. On constate que chaque compteur est initialisé avec une valeur égale à 1. On comprend que l'initialisation des compteurs pourrait se faire avec toute autre valeur, de préférence non nulle. Les genres et les états émotionnels renseignés dans la table de comptage peuvent être fixés de manière prédéterminée, ou bien être récupérés auprès d'un serveur externe, par exemple.

Comme précédemment, l'étape d'association E20 des genres (et éventuellement des sous-genres) à des états émotionnels est suivie d'une étape E40 de détection d'un état émotionnel courant de l'utilisateur 12. Cette étape E40 de détection d'un état émotionnel courant de l'utilisateur 12 peut par exemple mettre en oeuvre la technique de détection connue décrite en relation avec le premier mode de réalisation et qui consiste à détecter les émotions d'un individu à partir d'images ou à partir de la voix de l'utilisateur 12.

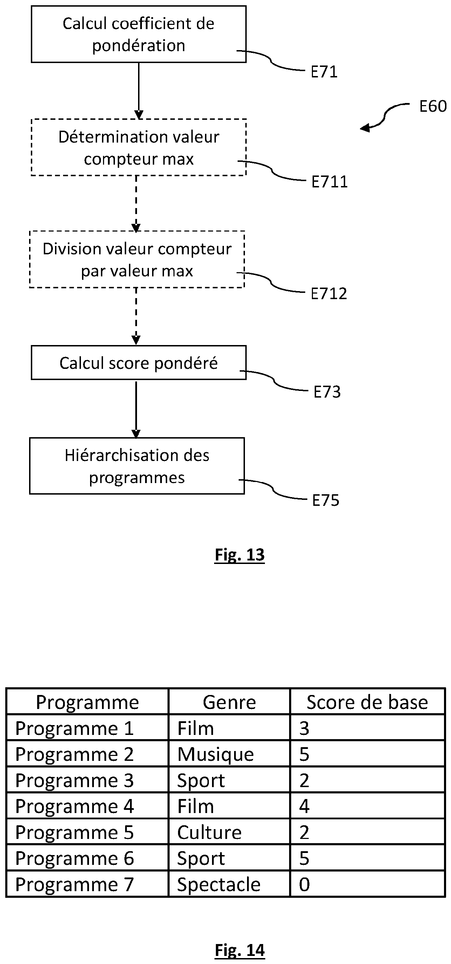

L'étape de réalisation d'un tri E60 des programmes du catalogue tenant au moins compte de l'état émotionnel détecté et des genres associés à l'état émotionnel détecté comprend les sous-étapes (illustrées sur la Fig. 13) de :
- calcul E71, par le décodeur audiovisuel 10, d'un coefficient de pondération pour chacun des genres associés à l'état émotionnel détecté, le calcul du coefficient de pondération comprenant :
   o pour l'état émotionnel détecté, la détermination E710, par le décodeur audiovisuel 10, d'une valeur maximale parmi les compteurs associés à chacun des genres ;
   o pour chaque genre associé à l'état émotionnel détecté, la division E712, par le décodeur audiovisuel 10, de la valeur du compteur correspondant par la valeur maximale déterminée ;
- pour chaque programme compris dans une liste de programmes proposés dans laquelle chaque programme présente un score prédéterminé et un genre, calcul E73, par le décodeur audiovisuel 10, d'un score pondéré par la multiplication du score prédéterminé du programme par le coefficient de pondération correspondant audit genre ;
- hiérarchisation E75, par le décodeur audiovisuel 10, des programmes de la liste de programmes proposés par ordre décroissant de score pondéré.

Dans ce deuxième mode de réalisation, le décodeur audiovisuel 10 reçoit une liste de programmes dans laquelle un score prédéterminé est associé à chaque programme de la liste (en plus des informations bibliographiques et de l'indication du genre (et éventuellement du sous-genre) classiquement reçues). Par exemple, le score prédéterminé comprend une note entre 0 et 5 correspondant à la popularité du programme auprès des autres utilisateurs. Le score prédéterminé peut être déterminé en tenant compte du nombre de lectures du programme (total ou sur une période déterminée), de l'âge du programme, de sa note moyenne donnée par les autres utilisateurs, de sa notation par les plateformes, de sa notation par les médias ou de tout autres critères. Une combinaison de ces critères peut également être utilisée pour déterminer la popularité du programme. Le score prédéterminé est indépendant de l'utilisateur et est fixé par des règles externes au procédé de l'invention, par exemple.

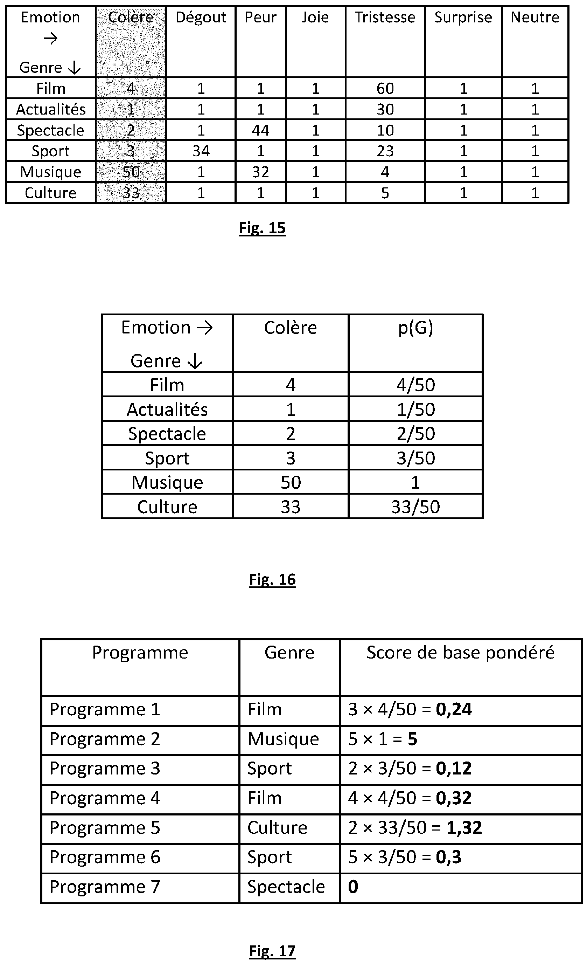

La Fig. 14 illustre un exemple de liste de programmes dans laquelle un score prédéterminé est associé à chaque programme. Afin de faciliter la compréhension de l'étape de réalisation d'un tri E60 desdits programmes dudit catalogue tenant au moins compte de l'état émotionnel détecté et des genres associés à l'état émotionnel détecté, la Fig. 15 illustre un exemple de table de comptage après plusieurs jours d'utilisation. On note que les compteurs de certains genres associés à certains états émotionnels ont été incrémentés. Afin de calculer à l'étape E71 le coefficient de pondération p(G), le décodeur audiovisuel 10 détermine à l'étape E711 tout d'abord la valeur maximale parmi les compteurs associés à chacun des genres. En d'autres termes, le décodeur audiovisuel 10 va parcourir la table de comptage et rechercher quel compteur, pour l'état émotionnel courant détecté, est le plus élevé. Si l'on prend comme exemple l'état émotionnel correspondant à la colère (« Anger » en anglais) dans la table de comptage de la Fig. 15, la valeur de compteur la plus élevée correspond au genre musique « Music » en anglais. La valeur maximale retenue est donc celle du compteur associé au genre musique pour l'état émotionnel de la colère, à savoir une valeur de compteur égale à 50.

Dans une variante, le coefficient de pondération pourrait être calculé, ou déterminé, différemment, par exemple en divisant la valeur du compteur correspondant par la somme des valeurs des compteurs associés à l'état émotionnel détecté, ou en prenant directement la valeur du compteur correspondant comme coefficient de pondération.

Ensuite, pour chaque genre associé à l'état émotionnel courant détecté, le décodeur audiovisuel 10 va diviser E712 la valeur de chaque compteur par la valeur maximale déterminée précédemment.

Dans cet exemple, et comme illustré sur la Fig. 16, les valeurs des compteurs de chaque genre associé à l'état émotionnel de la colère sont divisées par la valeur maximale déterminée, à savoir divisées par 50. Ainsi, le genre film (« Movie » en anglais) pour l'état émotionnel de la colère présente un coefficient de pondération P(G) de 4/50, correspondant à la valeur du compteur du genre film pour l'état émotionnel de la colère (soit une valeur de 4) divisée par la valeur maximale déterminée (soit 50).

Le coefficient de pondération P(G) est dans cet exemple calculé pour chaque genre correspondant à l'état émotionnel détecté.

Lorsque les coefficients de pondérations P(G) de chaque genre correspondant à l'état émotionnel détecté sont calculés, le décodeur audiovisuel 10 calcule E73 le score prédéterminé pondéré de chaque programme de la liste de programmes proposés récupérée par le décodeur audiovisuel 10.

Pour ce faire, le décodeur audiovisuel 10 multiplie le score prédéterminé de chaque programme par le coefficient de pondération correspondant au genre du programme.

Si l'on se réfère à l'exemple décrit ici, le tableau illustré sur la Fig. 17 montre le score pondéré de chaque programme de la liste de programmes de la Fig. 14. On note ainsi que le programme 1 présente un score pondéré de 0,24, correspondant à son score prédéterminé (à savoir 3) multiplié par le coefficient de pondération du genre film (à savoir 4/50).

Le calcul du score pondéré est effectué pour chaque programme de la liste de programmes proposée au décodeur audiovisuel 10. Le score pondéré permet donc de pondérer le score prédéterminé du programme en tenant compte de l'état émotionnel courant de l'utilisateur. Ensuite, les programmes de la liste de programmes proposée au décodeur audiovisuel 10 sont hiérarchisés E75 par ordre décroissant de score pondéré de sorte à proposer à l'utilisateur une liste de programmes qui tient compte de son état émotionnel courant.

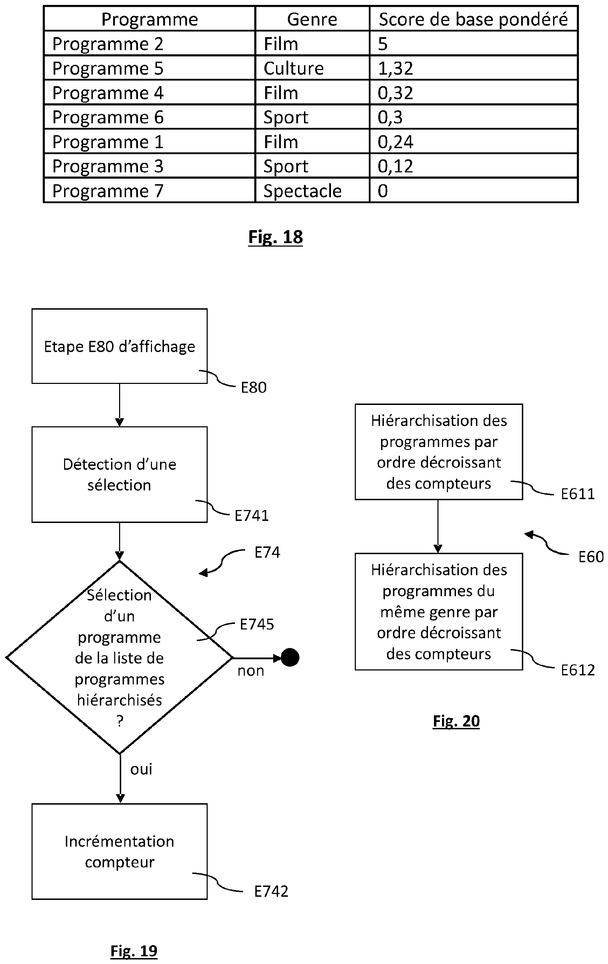

La Fig. 18 illustre donc la liste de programmes hiérarchisée par ordre décroissant de score pondéré. On note donc que, toujours pour l'état émotionnel de la colère, le décodeur audiovisuel 10 va proposer à l'utilisateur un programme du genre musique, puis un programme du genre culture, puis un programme du genre film, etc.

Dans ce mode de réalisation, le décodeur audiovisuel 10 trie donc les programmes proposés en s'appuyant sur un coefficient de pondération tenant compte de l'état émotionnel détecté de l'utilisateur. La liste de programmes affichée à l'utilisateur peut donc comprendre des programmes de genre et/ou sous-genre différents.

Lors de l'étape E80 d'affichage d'une liste d'au moins un programme audiovisuel tenant compte du tri desdits programmes du catalogue, le décodeur audiovisuel 10 va afficher la liste de programmes hiérarchisés décrite ci-dessus.

Le procédé 100 comprend en outre, dans ce deuxième mode de réalisation, une étape E74 exécutée par le décodeur 10 de mise à jour de la table de comptage. Pour ce faire, et comme illustré sur la Fig. 19, suite à l'étape E80 d'affichage de la liste d'au moins un programme audiovisuel tenant compte du tri des programmes du catalogue, le décodeur audiovisuel 10 va détecter à l'étape E741 une sélection de l'utilisateur.

À l'étape E745 le décodeur audiovisuel 10 vérifie si l'utilisateur a sélectionné un des programmes de la liste de programmes hiérarchisés.

Dans la négative, le décodeur audiovisuel 10 interrompt le présent algorithme. Dans l'affirmative, le décodeur audiovisuel 10 met à jour la table de comptage en incrémentant à l'étape E742 d'une valeur le compteur correspondant au genre du programme sélectionné pour l'état émotionnel courant détecté.

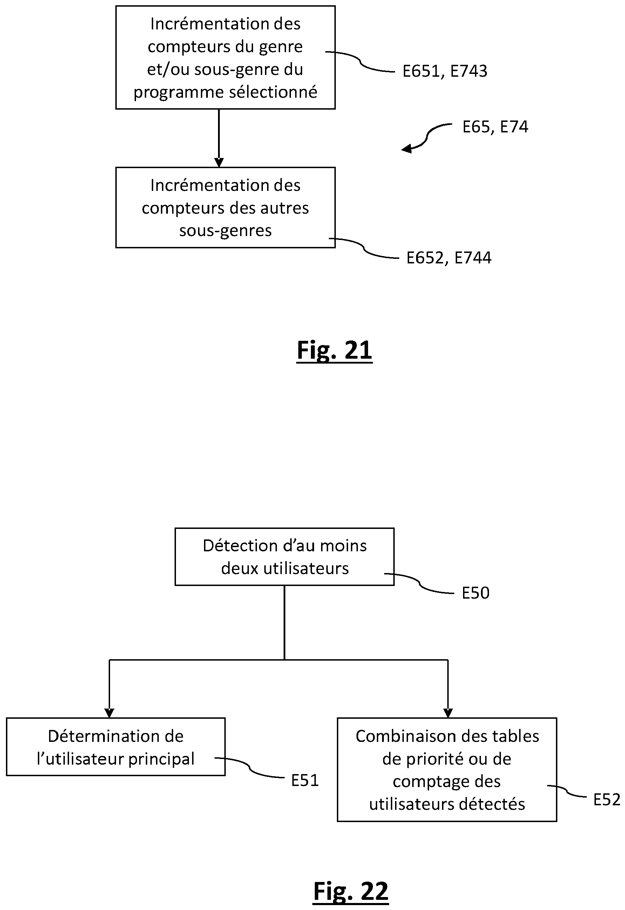

Dans une variante de ce deuxième mode de réalisation, le décodeur audiovisuel 10 ne calcule pas de score pondéré à l'étape de sélection E60 d'au moins un genre à partir au moins de l'état émotionnel détecté. Selon cette variante et comme illustré sur la Fig. 20, l'étape d'un tri E60 des programmes du catalogue tenant au moins compte de l'état émotionnel détecté et des genres associés à l'état émotionnel détecté comprend les sous-étapes de :
- hiérarchisation E611, par le décodeur audiovisuel 10, des programmes de la liste de programmes proposés, dans laquelle chaque programme présente un score prédéterminé, par ordre décroissant du compteur associé au genre et à l'état émotionnel détecté ; puis
- hiérarchisation E612, par le décodeur audiovisuel 10, des programmes de la liste de programmes ayant le même genre par ordre décroissant du score prédéterminé. Dans une autre variante, compatible avec les deux modes de réalisation de l'invention décrits ci-dessus ainsi que leurs variantes, chaque genre comprend au moins un sous-genre. Les tables de priorité et de comptage comprennent donc un compteur pour chacun des sous-genres, en complément ou à la place des compteurs associés à chaque genre. Ainsi, et comme illustré sur la Fig. 21, l'étape de mise à jour E65 de la table de priorité et l'étape de mise à jour E74 de la table de comptage comprennent chacune les sous-étapes de, lorsque l'au moins un utilisateur visionne un des programmes recommandés :

- incrémentation E651, E743 des compteurs du genre et du sous-genre du programme sélectionné par l'au moins un utilisateur correspondant à l'état émotionnel détecté d'une première valeur ;
- incrémentation E652, E744 des compteurs des autres sous-genres associés au genre du programme sélectionné par l'au moins un utilisateur correspondant à l'état émotionnel détecté d'une deuxième valeur.

De cette manière, il est possible de prendre en compte le genre et/ou le sous-genre d'un programme afin d'effectuer une recommandation optimale à l'utilisateur en fonction de son état émotionnel courant détecté.

Dans un exemple non illustré, le décodeur audiovisuel 10 n'incrémente que les compteurs de sous-genres. Ainsi, le décodeur audiovisuel 10 incrémente d'une première valeur égale à deux la valeur du compteur correspondant au couple constitué du genre principal et du sous-genre du programme sélectionné par l'utilisateur, tandis que le décodeur audiovisuel 10 incrémente d'une deuxième valeur égale à un la valeur des compteurs correspondant au genre principal mais pas au sous-genre du programme choisi.

Dans cet exemple, si l'on considère que l'utilisateur sélectionne un film comique proposé par le procédé, le film comique est un programme appartenant au genre principal « film » et au sous-genre « film / comédie). Alors, le compteur du sous-genre « film / comédie » est incrémenté d'une première valeur égale à 2, tandis que les compteurs des autres sous-genres du genre « film » (comme par exemple les sous-genres « film / drame, » « film / science-fiction, » « film / aventure, » etc.) ne sont incrémentés que d'une deuxième valeur égale à 1.

Dans l'exemple décrit ci-dessus, on ne considère que les compteurs des sous-genres. On comprend aisément que le nombre de lectures du genre peut être obtenu en incrémentant le compteur correspondant ou en déterminant sa valeur à partir de la somme des compteurs des sous-genres qui lui sont associés, par exemple.

Il convient de noter que plusieurs genres peuvent avoir des sous-genres similaires, par exemple il peut y avoir un genre « théâtre » avec des sous-genres similaires à ceux du genre « film » de l'exemple précédent, tels que « théâtre / comédie, » « théâtre / drame, » etc. Dans un mode de réalisation préféré de l'invention, les sous-genres de genres distincts sont eux-mêmes considérés comme distincts. Ainsi par exemple le sous-genre « film / comédie » et le sous-genre « théâtre / comédie » ont chacun un compteur distinct. Selon un mode alternatif de réalisation les sous-genres similaires utilisent le même compteur, de sorte qu'il y a un même compteur commun pour les sous-genres « film / comédie, » « théâtre / comédie, » « série / comédie, » etc.

Ainsi, le procédé selon l'invention permet d'améliorer le ciblage des programmes recommandés à l'utilisateur.

Il est à noter que le procédé 100 de recommandation peut être déclenché de différentes façons. Pour ce faire, le procédé 100 peut comprendre une étape E101 de lancement/déclenchement dudit procédé 110 :
- à la mise en route du décodeur audiovisuel 10 ; ou
- à la détection par le décodeur de la présence d'au moins un utilisateur par le décodeur audiovisuel 10 ; ou
- à la détection par le décodeur d'une sélection/recherche d'un programme par l'au moins un utilisateur.

Il est à noter que, dans le cas où il y a plusieurs utilisateurs dans un même foyer, chacun des utilisateurs peut avoir sa propre table de priorité et/ou de comptage. Dans ce cas, le procédé 100 de recommandation peut comprendre une étape d'identification, par le décodeur audiovisuel 10, de l'utilisateur. Par exemple, l'identification de l'utilisateur peut se faire manuellement via le dispositif de commande 121 ou automatique via les moyens de détection 206 de l'état émotionnel. En effet, les moyens de détection 206 peuvent être utilisés, outre que pour la détection de l'état émotionnel courant de l'utilisateur, pour effectuer une identification de l'utilisateur, par exemple par une reconnaissance visuelle ou par une reconnaissance audio de la voix de l'utilisateur. Des techniques de l'art antérieur permettent d'effectuer une telle identification et ne sont donc pas décrites en détails ici.

Il est à noter que, de manière optionnelle, le procédé 100 de recommandation peut comprendre une étape de détection E50, par le décodeur audiovisuel 10, du nombre d'utilisateurs orientés vers les moyens de détection 206. Dans ce cas, le procédé 100 de recommandation peut soit prendre en compte un seul des utilisateurs, ou soit prendre en prendre en compte deux ou plus d'utilisateurs pour effectuer la recommandation.

Pour ce faire, et comme illustré sur la figure 22, en cas de détection E50 d'au moins deux utilisateurs, le procédé 100 de recommandation comprend en outre :
- une sous-étape de détermination E51, par le décodeur audiovisuel 10, de l'utilisateur principal sur la base d'au moins un critère prédéterminé, la sous-étape d'utilisation E21d'une table de priorité ou à la sous-étape d'utilisation E31 d'une table de comptage s'effectuant à partir de ladite table de priorité ou de ladite table de comptage associée audit utilisateur principal déterminé ; ou
- une sous-étape de combinaison E52, par le décodeur audiovisuel 10, des tables de priorité ou de comptage de chacun des utilisateurs détectés, la sous-étape d'utilisation E21 d'une table de priorité ou E31 d'une table de comptage s'effectuant à partir de la table de priorité ou de la table de comptage issue de cette combinaison.

Ainsi, selon le premier cas, le décodeur audiovisuel 10 détermine à l'étape E51 un utilisateur principal parmi les utilisateurs détectés de sorte à ne prendre en compte que la table de priorité ou de comptage de cet utilisateur principal. Cette sous-étape E51 de détermination de l'utilisateur principal peut par exemple s'appuyer sur l'un ou plusieurs des critères suivants : l'ancienneté de la table de priorité ou de comptage, un compteur d'heures de visionnage de programmes le plus élevé, une déclaration manuelle de la part des utilisateurs, etc.

Dans ce premier cas, n'est pris en compte pour la réalisation d'un tri des programme du catalogue, que la table de priorité ou de comptage de l'utilisateur déterminé comme étant l'utilisateur principal. Il est également envisageable de ne prendre en compte que l'état émotionnel de l'utilisateur déterminé comme étant l'utilisateur principal pour effectuer la recommandation.

Selon le deuxième cas, le décodeur audiovisuel 10 combine E51 les tables de priorité ou de comptage de chacun des utilisateurs détectés. Pour ce faire, le décodeur audiovisuel 10 peut, par exemple, additionner les compteurs des différents utilisateurs relatifs à chaque genre associé à chaque état émotionnel pour n'obtenir qu'une seule table de priorité ou de comptage, dite table combinée. Ainsi, cette table de priorité ou de comptage combinée ne comptera qu'un seul compteur par genre donné associé à un état émotionnel donné, la valeur de ce compteur représentant la somme des compteurs de chaque utilisateur pour le genre donné associé à l'état émotionnel donné.

Selon une autre approche (compatible avec le premier mode de réalisation uniquement), le décodeur audiovisuel 10 peut, par exemple, combiner les tables de priorité des différents utilisateurs en prenant en compte les compteurs de chacun des utilisateurs dans une même table de priorité (et en réorganisant les compteurs selon l'ordre de priorité). Ainsi, les préférences de chaque utilisateur peuvent être prises en compte, sans discrimination des utilisateurs secondaires par rapport à un utilisateur principal.

Dans les deux cas, il est possible de mettre à jour les tables de priorité ou de comptage de chacun des utilisateurs, soit de manière individuelle (par exemple lorsque la table utilisée est celle de l'utilisateur principal) soit de manière collective (c'est-à-dire que la table de priorité ou de comptage de chacun des utilisateurs peut être mise à jour en fonction du programme sélectionné).

Il est également possible, lorsque l'on souhaite prendre en compte l'état émotionnel de chaque utilisateur, de mélanger les tables de priorité des différents utilisateurs et de présenter une ou plusieurs listes de programmes qui tiennent compte de l'état émotionnel des différents utilisateurs.

Selon une autre approche (compatible avec le second mode de réalisation uniquement), le décodeur audiovisuel 10 détecte l'état émotionnel courant de chaque utilisateur présent et calcule un coefficient de pondération pour chaque utilisateur à partir de l'état émotionnel courant détecté et de la table de comptage de chaque utilisateur. Ensuite le décodeur audiovisuel 10 calcule un score pondéré pour chaque programme en multipliant le score prédéterminé par les coefficients de pondération de tous les utilisateurs présents correspondant au genre (et/ou au sous-genre éventuellement) du programme. Ainsi la recommandation tient également compte de l'état émotionnel de chaque utilisateur.

Il est à noter que, lors de l'initialisation du décodeur audiovisuel 10, la création des tables de priorité et/ou de comptage par le décodeur audiovisuel 10 peut prendre en compte des préférences de l'utilisateur concernant la recommandation de programmes. Par exemple, il est possible de proposer un questionnaire à l'utilisateur de sorte à tenir compte de ses préférences tant qu'un nombre prédéterminé de lectures n'est pas atteint pour que le procédé de recommandation propose des programmes particulièrement pertinents basés sur les compteurs de chaque genre associé à chaque état émotionnel.

Par exemple, le questionnaire proposé par le décodeur audiovisuel 10 peut comprendre des questions du type :
- « Quels genres de films par priorité aimez-vous regarder lorsque vous êtes joyeux ? », de sorte que l'utilisateur soit invité à sélectionner son premier choix, puis son second, et ainsi de suite.
- « Aimeriez-vous regarder un programme de divertissement lorsque vous êtes triste ? », de sorte que l'utilisateur soit invité à répondre par oui ou non.

Selon une autre approche, lors de l'initialisation du décodeur audiovisuel 10, la création des tables de priorité et/ou de comptage par le décodeur audiovisuel 10 peut prendre en compte des informations provenant d'une source externe, comme par exemple des informations fournies par l'opérateur via le réseau 20.

Ainsi, au lieu de débuter avec une table de priorité vide ou une table de comptage dans laquelle tous les compteurs sont initialisés avec la valeur 1, les tables de priorité et de comptage peuvent être préremplies, par exemple à partir d'une moyenne sur l'ensemble des nombres de lectures d'un parc d'abonnés. Ensuite, le processus d'apprentissage (c'est-à-dire de mise à jour des tables au fur et à mesure des sélections de l'utilisateur) peut se poursuivre comme décrit précédemment afin d'adapter les tables à chaque utilisateur. Les tables de priorité et/ou de comptage à l'initialisation du décodeur audiovisuel 10 peuvent être transmises par le réseau TCP/IP ou bien par une table ou un descripteur privé étendant la norme DVB dans une transmission RF.

Optionnellement, les tables de priorité et/ou de comptage à l'initialisation du décodeur audiovisuel 10 peuvent être liées à un profil général du genre, par exemple ; « profil Film », « profil Sport » qui serait choisi par l'utilisateur lors de l'initialisation du décodeur. Optionnellement, les tables de priorité et/ou de comptage à l'initialisation du décodeur audiovisuel 10 peuvent être liées à un profil de l'utilisateur lié à son âge et/ou son sexe, ou tout autre critère.

## Revendications

1. Procédé (100) de recommandation d'une liste d'au moins un programme audiovisuel parmi un catalogue de programmes à au moins un utilisateur (12) orienté vers un décodeur audiovisuel (10) et des moyens d'affichage (13), lesdits programmes dudit catalogue étant catégorisés en genres, **caractérisé en ce que** le procédé (100) comprend les étapes exécutées par ledit décodeur audiovisuel (10) de :
- association (E20) desdits genres à des états émotionnels ;
- détection (E40) d'un état émotionnel courant dudit au moins un utilisateur ;
- réalisation d'un tri (E60) desdits programmes dudit catalogue de programmes tenant au moins compte dudit état émotionnel détecté et desdits genres associés audit état émotionnel détecté ;
- affichage (E80) d'une liste d'au moins un programme audiovisuel tenant compte dudit tri desdits programmes dudit catalogue de programmes, ;
dans lequel ladite étape d'association (E20) desdits genres auxdits états émotionnels comprend :
- soit une sous-étape d'utilisation (E21) d'une table de priorité dans laquelle, pour chacun desdits états émotionnels, lesdits genres sont classés selon un ordre de priorité, ledit classement tenant au moins compte d'un compteur de lectures de chaque genre associé à chaque état émotionnel, ladite table de priorité comprenant en outre un genre par défaut attribué en dernier selon l'ordre de priorité pour chacun desdits états émotionnels ;
- soit une sous-étape d'utilisation (E31) d'une table de comptage comprenant un compteur de lectures par l'utilisateur pour chaque genre associé à un état émotionnel.

2. Procédé (100) selon la revendication 1, dans lequel ladite étape de réalisation d'un tri (E60) desdits programmes dudit catalogue tenant au moins compte dudit état émotionnel détecté et desdits genres associés audit état émotionnel détecté comprend une sous-étape de :
- pointage (E61), dans ladite table de priorité, dudit genre classé en premier selon ledit ordre de priorité pour ledit état émotionnel détecté, et
- détection (E62) d'une sélection par l'utilisateur et si l'utilisateur ne sélectionne pas un programme correspondant audit premier genre pointé, pointage (E63) du genre suivant dans la table de priorité jusqu'à ce que l'utilisateur sélectionne un programme dudit genre pointé ou jusqu'à ce que ledit genre pointé soit ledit genre par défaut (E64).

3. Procédé (100) selon la revendication 1, dans lequel ladite étape d'affichage (E80) de ladite liste d'au moins un programme audiovisuel tenant compte dudit tri desdits programmes dudit catalogue comprend les sous-étapes de :
- ordonnancement (E81) dudit au moins un programme de ladite liste d'au moins un programme à afficher, ledit ordonnancement tenant au moins en partie compte d'un critère de priorité dudit au moins un programme ; et
- affichage (E82) de ladite liste d'au moins un programme en fonction dudit ordonnancement.

4. Procédé (100) selon la revendication 1, dans lequel ladite étape de réalisation d'un tri (E60) desdits programmes dudit catalogue tenant au moins compte dudit état émotionnel détecté et desdits genres associés audit état émotionnel détecté comprend les sous-étapes de :
- calcul (E71) d'un coefficient de pondération (P(G)) pour chacun desdits genres associés audit état émotionnel détecté ;
- pour chaque programme compris dans une liste de programmes proposés dans laquelle chaque programme présente un score prédéterminé et un genre, calcul (E73) d'un score pondéré par la multiplication dudit score prédéterminé dudit programme par ledit coefficient de pondération (P(G)) correspondant audit genre ;
- hiérarchisation (E75) desdits programmes de ladite liste de programmes proposés par ordre décroissant de score pondéré.

5. Procédé (100) selon la revendication 4, dans lequel la sous-étape de calcul (E71) d'un coefficient de pondération (P(G)) pour chacun desdits genres associés audit état émotionnel détecté comprend :
∘ pour ledit état émotionnel détecté, la détermination (E711) d'une valeur maximale parmi lesdits compteurs associés à chacun desdits genres ;
∘ pour chaque genre associé audit état émotionnel détecté, la division (E712) de ladite valeur dudit compteur correspondant par ladite valeur maximale déterminée.

6. Procédé (100) selon la revendication 1, dans lequel ladite étape de réalisation d'un tri (E60) desdits programmes dudit catalogue tenant au moins compte dudit état émotionnel détecté et desdits genres associés audit état émotionnel détecté comprend une sous-étape de :
- hiérarchisation des programmes d'une liste de programmes proposés, dans laquelle chaque programme présente un score prédéterminé, par ordre décroissant dudit compteur associé audit genre et audit état émotionnel détecté ; puis
- hiérarchisation desdits programmes de ladite liste de programmes ayant le même genre par ordre décroissant du score prédéterminé.

7. Procédé (100) selon la revendication 1, dans lequel il comprend une étape de mise à jour (E65, E74) de ladite table de priorité ou de ladite table de comptage, lorsque ledit au moins un utilisateur lit pendant au moins une durée prédéterminée un desdits programmes recommandés, par incrémentation d'une valeur dudit compteur associé audit programme lu par ledit au moins un utilisateur.

8. Procédé (100) selon la revendication 7, dans lequel chaque genre comprend au moins un sous-genre et dans lequel ladite table de priorité et ladite table de comptage comprennent un compteur de lectures de chaque genre et un compteur de lectures de chaque sous-genre,
l'étape de mise à jour (E65, E74) de ladite table de priorité et de ladite table de comptage comprenant les sous-étapes de, lorsque ledit au moins un utilisateur lit un desdits programmes recommandés :
- incrémentation (E651, E743) desdits compteurs du genre et du sous-genre du programme sélectionné par ledit au moins un utilisateur correspondant audit état émotionnel détecté d'une première valeur ;
- incrémentation (E652, E744) des compteurs des autres sous-genres associés au genre du programme sélectionné par ledit au moins un utilisateur correspondant audit état émotionnel détecté d'une deuxième valeur.

9. Procédé (100) selon la revendication 1, dans lequel, lorsque ledit décodeur audiovisuel (10) détecte (E50) au moins deux utilisateurs, le procédé (100) comprend :
- une étape de détermination (E51) de l'utilisateur principal sur la base d'au moins un critère prédéterminé, ladite sous-étape d'utilisation (E21) d'une table de priorité ou (E31) d'une table de comptage s'effectuant à partir de ladite table de priorité ou de ladite table de comptage associée audit utilisateur principal déterminé ; ou
- une étape de combinaison (E52) desdites tables de priorité ou de comptage de chacun desdits utilisateurs détectés, ladite sous-étape d'utilisation (E21) d'une table de priorité ou (E31) d'une table de comptage s'effectuant à partir de la table de priorité ou de la table de comptage issue de cette combinaison.

10. Procédé (100) selon l'une quelconque des revendications 1 à 9, **caractérisé en ce qu'**il comprend une étape (E101) de déclenchement dudit procédé (100) :
- à la mise en route dudit décodeur audiovisuel (10) ; ou
- à la détection d'au moins un utilisateur par ledit décodeur audiovisuel (10) ; ou
- à la sélection/recherche d'un programme par ledit au moins un utilisateur.

11. Décodeur audiovisuel (10) pour la mise en oeuvre du procédé (100) de recommandation d'une liste d'au moins un programme audiovisuel parmi un catalogue de programmes à au moins un utilisateur selon l'une quelconque des revendications 1 à 10, lesdits programmes dudit catalogue étant catégorisés en genres, ledit décodeur audiovisuel (10) étant destiné à être connecté à une base de données distante, un moyen d'affichage (13) et un dispositif de commande (121) utilisable par un utilisateur (12) pour commander ledit décodeur audiovisuel (10), ledit décodeur (10) comprenant une circuiterie électronique adaptée et configurée pour :
- associer lesdits genres à des états émotionnels ;
- détecter un état émotionnel courant dudit au moins un utilisateur ;
- réaliser un tri desdits programmes dudit catalogue tenant au moins compte dudit état émotionnel détecté et desdits genres associés audit état émotionnel détecté ;
lesdits moyens d'affichage (13) étant destinés à afficher une liste d'au moins un programme audiovisuel tenant compte dudit tri desdits programmes dudit catalogue ; dans lequel l'association desdits genres auxdits états émotionnels comprend :
- soit l'utilisation d'une table de priorité dans laquelle, pour chacun desdits états émotionnels, lesdits genres sont classés selon un ordre de priorité, ledit classement tenant au moins compte d'un compteur de lectures de chaque genre associé à chaque état émotionnel, ladite table de priorité comprenant en outre un genre par défaut attribué en dernier selon l'ordre de priorité pour chacun desdits états émotionnels ;
- soit l'utilisation d'une table de comptage comprenant un compteur de lectures par l'utilisateur pour chaque genre associé à un état émotionnel.

12. Un produit programme d'ordinateur **caractérisé en ce qu'**il comprend des instructions pour implémenter, par un décodeur audiovisuel, le procédé selon l'une quelconque des revendications 1 à 10, lorsque ledit programme est exécuté par un processeur du décodeur audiovisuel.

13. Un support de stockage **caractérisé en ce qu'**il stocke un programme d'ordinateur comprenant des instructions pour implémenter, par un décodeur audiovisuel, le procédé selon l'une quelconque des revendications 1 à 10, lorsque ledit programme est exécuté par un processeur du décodeur audiovisuel.
